# EUROPÄISCHE PATENTSCHRIFT

(11) **EP 3 174 481 B1**
(45) Veröffentlichungstag und Bekanntmachung des Hinweises auf die Patenterteilung: **26.12.2018**
(21) Anmeldenummer: 15766737.9
(22) Anmeldetag: 29.07.2015
(51) Int. Cl.: A61B 17/50

(54) **GREIFERKOPF ZUM ERGREIFEN EINER ZECKE UND ZECKENGREIFER**
GRIPPER HEAD FOR GRABBING A TICK AND TICK GRIPPER
TÊTE DE PRÉHENSION DE TIQUE ET DISPOSITIF DE PRÉHENSION DE TIQUE

(30) Priorität: 30.07.2014 DE 202014006075 U
(43) Veröffentlichungstag der Anmeldung: 07.06.2017
(73) Patentinhaber: Meinhold, Matthias, 90402 Nürnberg (DE)
(72) Erfinder: MEINHOLD, Matthias, 90402 Nürnberg (DE); Zimmermann, Frédéric, 90482 Nürnberg (DE); HAMM, Wolfgang, 57015 Bretten (DE)
(74) Vertreter: Meyer, Enno
(86) Internationale Anmeldenummer: PCT/DE2015/000374
(87) Internationale Veröffentlichungsnummer: WO 2016/015702

(56) Entgegenhaltungen:
- DE-A1- 19 652 218
- DE-A1- 19 860 172
- DE-A1-102004 031 682

## Beschreibung

Die Erfindung betrifft einen Greiferkopf zum Ergreifen einer Zecke gemäß dem Oberbegriff des Anspruchs 1 sowie einen Zeckengreifer mit einem solchen Greiferkopf gemäß dem Oberbegriff des Anspruchs 7.

Von Zecken auf den Menschen und das Tier übertragene Erkrankungen breiten sich immer weiter aus, wobei je nach Infektion der Zecke verschiedene Erkrankungen übertragen werden können. Die Borreliose ist die häufigste durch Zecken übertragene Erkrankung in Deutschland mit geschätzten 60.000 - 100.000 Neuerkrankungen pro Jahr, wobei in Deutschland bis zu 30 % der Zecken Borrelien enthalten. An Frühsommer-Meningoenzephalitis (FSME) erkranken allein in Deutschland jährlich etwa 250 Personen, rund 30 Prozent davon schwer. Zehn Prozent der erkrankten Patienten weisen bleibende, teilweise schwerste neurologische Schäden auf. Zwei Prozent der Patienten sterben daran.

Die Zunahme zeckenübertragener Krankheiten ist besorgniserregend. 80 Prozent aller Infektionen sind Schätzungen zufolge Ergebnis einer unsachgemäßen Entfernung der Zecke. Die rechtzeitige und sachgemäße Entfernung der Zecke ist Vorbeugung und Behandlung zugleich und kann dazu beitragen, die Zahl der Neuerkrankungen bei Mensch und Tier zu senken. Unter sachgemäßer Entfernung wird das Entfernen der unbeschädigten Zecke verstanden, ohne dass sie gequetscht, anderweitig irritiert und eine Infektion dadurch erst verursacht wird.

Herkömmlicherweise werden Zecken mit einer Zange, Karte, Schlinge, Pinzette oder mit den Fingernägeln entfernt. Doch es sollte darauf geachtet werden, die Zecke auf keinen Fall zu quetschen, denn in den Speicheldrüsen und im Magen-Darm-Trakt können sich Krankheitserreger befinden, die bei der geringsten Druckausübung auf den Menschen übertragen werden. Daher sind starre Materialien, die den Zeckenkörper unweigerlich quetschen und damit das Erkrankungsrisiko wesentlich erhöhen, zu meiden.

Neuere wissenschaftliche Untersuchungen zeigen, dass bei Betrachtung aller Kriterien den Geräten, welche die Zecken mittels Rotation entfernen gegenüber jenen, welche die Zecken durch Zug entfernen, der Vorzug gegeben werden kann. So in Klaus Robisch: "Tick Removal - Vergleich von fünf verschiedenen Zeckenentfernungsgeräten", Diplomarbeit zur Erlangung der Würde eines Diplomtierarztes der Veterinärmedizinischen Universität Wien, Wien, November 2010 (http://www.zeckenzange.eu/Zeckenentfernung.pdf).

Aus der Druckschrift DE 10 2004 031 682 A1 ist eine Vorrichtung zum Entfernen von Zecken oder ähnlichen Parasiten aus der Haut von Tieren und Menschen bekannt, die auch als Zeckengreifer bezeichnet wird. Dabei weist die Vorrichtung ein Gehäuse, einen spreizbaren Greiferkopf, eine Spreizvorrichtung zum Spreizen des Greiferkopfes und eine Drehvorrichtung zur Drehen des Greiferkopfes um die Längsachse der Vorrichtung auf, wobei der Greiferkopf in seinem ungespreizten Zustand einen im wesentlichen geschlossenen Hohlraum zur Aufnahme des Parasiten bzw. der Zecke umschließt. Weiterhin weist die Vorrichtung oder Zeckengreifer eine in axialer Richtung wirkende Druckvorrichtung auf, mittels der die Spreizung und Drehung des Greiferkopfes bewirkt wird.

Bei dem bekannten Zeckengreifer kann es zu einem "Ausleihern" des Greiferkopfes nach fortgesetzter Betätigung kommen, so dass ein exakter Schluss der Greifbacken nicht unbedingt gewährleistet ist.

Die Druckschrift DE 196 52 218 A1 betrifft eine Zeckenzange, bestehend aus zwei sich kreuzenden Zangenarmen, welche an ihren freien Enden mit Greifbacken versehen und an ihren anderen Enden miteinander verbunden sind, einem von den Greifbacken gebildeten Maul, mit dem ein Zeckenkörper greifbar ist, und an den Greifbacken vorgesehenen, unter Federvorspannung aneinanderliegenden Greifkanten, die gegen die Federvorspannung zu öffnen sind, wobei sich die beiden Zangenarme in einem Federbereich derselben aufeinander zu biegen. Die beiden Zangenarme bilden die Zeckenzange als einstückiges Teil aus. Der Federbereich ist als gemeinsamer Federbereich an den beiden Enden der Zangenarme ausgebildet, an denen die Zangenarme miteinander verbunden sind.

Der Erfindung liegt daher die Aufgabe zugrunde, den Greiferkopf eines Zeckengreifers und damit den Zeckengreifer selbst zu verbessern.

Diese Aufgabe wird durch einen Greiferkopf für einen Zeckengreifer mit den Merkmalen des Anspruchs 1 sowie durch einen entsprechenden Zeckengreifer mit den Merkmalen des Anspruchs 7 gelöst. Bevorzugte Ausgestaltungen der Erfindung sind Gegenstand der Unteransprüche.

Der erfindungsgemäße Greiferkopf zum Ergreifen und Fixieren einer Zecke umfasst zwei gegenüberliegende Greifbacken, wobei der Greiferkopf einen Zustand mit geöffneten Greifbacken und einen Zustand mit geschlossenen Greifbacken einnehmen kann. Weiter umfasst der Greiferkopf eine Halterung mit einer Schließfeder zur Aufnahme der beiden Greifbacken, wobei die Greifbacken so ausgestaltet sind, dass sie jeweils eine gekrümmte Außenseite und eine plane Innenseite aufweisen, die Schließfeder auf die beiden Greifbacken wirkt, und im geschlossenen Zustand des Greiferkopfes die planen Innenseiten der beiden Greifbacken aufeinander liegen, so dass ein geschlossener Körper gebildet wird.

Dabei weist die Schließfeder zwei gegenüber angeordnete Federelemente auf. Ferner weist jede Greifbacke auf Ihrer Außenseite eine Aussparung auf, so dass die Federelemente in die Aussparungen der Greifbacken eingreifen kann.

Vorzugsweise weisen die beiden Greifbacken jeweils eine innere Kavität auf. Durch die beiden Kavitäten der beiden Greifbacken wird im geschlossenen Zustand des Greiferkopfes ein innerer Hohlraum gebildet, der Teile des Zeckenkörpers aufnimmt, wodurch ein Druck auf den Zeckenkörper vermieden wird und die Zecke nur an ihrem Kopfteil von dem spitzen vorderen Teil des durch die Greifbacken gebildeten Körpers fixiert wird, wobei der geschlossenen Körper in etwa die Form eines nach vorne spitz zulaufenden Rotationsellipsoid hat.

Weiter bevorzugt weisen die Federelemente der Schließfeder eine vorgegebene Vorspannung auf, wodurch der Schluss der Greifbacken im geschlossenen Zustand des Greiferkopfes bewirkt wird. Dabei wird vorzugsweise die Federkonstante der Schließfeder so gewählt wird, dass keine übermäßige Quetschung der zu entfernenden Zecke erfolgt.

Vorzugsweise bestehen die Greifbacken aus einem thermoplastischen Elastomer (TPE). TPE gilt als Alternativwerkstoff zu Silikon und verfügt über exzellente mechanische Eigenschaften, denn es ist extrem flexibel, hat eine glatte Oberfläche, ist UV- und witterungsbeständig, besitzt ein gutes Rückstellvermögen und ist in einem weiten Temperatureinsatzbereich von - 40°C bis +120°C einsetzbar,

Weiter bevorzugt weist die Halterung des Greiferkopfes eine Einrichtung zur lösbaren Fixierung des Greiferkopfes an einem Zeckengreifer auf. Dies kann beispielsweise durch eine geeignete Steckverbindung realisiert sein, mittels der der Greiferkopf fest aber lösbar an den Zeckengreifer aufgesteckt werden kann.

Der erfindungsgemäße Zeckengreifer zum Ergreifen und Fixieren einer Zecke umfasst einen länglichen Korpus, eine in dem Korpus angeordnete Drück-/Dreheinrichtung und einen Greiferkopf zum Ergreifen und Fixieren der Zecke, wobei der Greiferkopf durch die an einem Ende aus dem Korpus herausragende Drück-/Dreheinrichtung in Rotation versetzt sowie geöffnet und geschlossen werden kann. Dabei kommt in dem Zeckengreifer ein im Vorangegangenen beschriebener Greiferkopf zum Einsatz.

Vorzugsweise ist die Drück-/Dreheinrichtung des Zeckengreifers an einem Ende des Korpus als Druckstift ausgebildet ist und an dem anderen Ende weist sie eine Aufnahmevorrichtung zum lösbaren Befestigen und Fixieren des Greiferkopfes auf.

Weiter bevorzugt wird mit dem Einfahren des Druckstiftes der Drück-/Dreheinrichtung in den Korpus der Greiferkopf zuerst in Rotation versetzt, kurz vor Erreichen der komplett eingefahrenen Position wird die Rotation beendet und der Greiferkopf geöffnet, mit dem Ausfahren des Druckstiftes aus dem Korpus wird der Greiferkopf wiederum geschlossen und bei einem weiteren Ausfahren des Druckstiftes aus dem Korpus wird der Greiferkopf in Rotation versetzt, bis der vollkommen ausgefahrenen Zustand des Druckstiftes erreicht ist.

Eine bevorzugte Ausführungsform der Erfindung wird nachfolgend anhand der Zeichnungen näher erläutert. Dabei zeigt
- Fig.1: einen Zeckengreifer mit Greiferkopf,
- Fig. 2: den geöffneten Greiferkopf bei einer Anwendung,
- Fig. 3: den geschlossenen Greiferkopf mit einer Zecke,
- Fig. 4: den Greiferkopf im Detail,
- Fig. 5: die Halterung des Greiferkopfes mit Schließfeder,
- Fig. 6: die Schließfeder im Detail,
- Fig. 7: eine Greifbacke des Greiferkopfes mit kleiner Kavität, und
- Fig. 8: eine Greifbacke des Greiferkopfes mit großer Kavität.

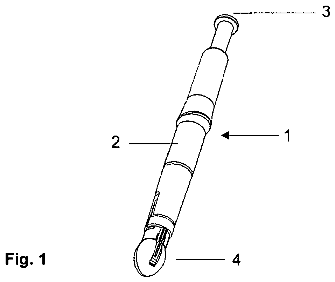

Fig. 1 zeigt einen Zeckengreifer 1 in perspektivischer Ansicht. Dabei umfasst der Zeckengreifer 1 einen Korpus 2, in dem eine Druck-/Dreheinrichtung 3 beweglich angeordnet ist, die auf einen Greiferkopf 4 wirkt. Dabei dient der Greiferkopf 4, der an dem Korpus 2 des Zeckengreifers 1 angeordnet ist, zum Ergreifen eines Parasiten, insbesondere einer Zecke, wobei der Greiferkopf 4 mit der Druck-/Dreheinrichtung 3 fest aber lösbar verbunden ist, beispielsweise mittels einer Rasteinrichtung.

Das dem Zeckengreifer 1 zugrunde liegende Konzept ist in der oben genannten DE 10 2004 031 682 A1 beschrieben. Durch einen axialen Druck, beispielsweise durch den Daumen einer Hand, auf die Druck-/Dreheinrichtung 3 in Richtung des Korpus 2 wird zuerst eine Rotation des Greiferkopfes 4 bewirkt. Kurz vor Erreichen der vollkommen eingefahrenen Position der Druck-/Dreheinrichtung 3 in den Korpus 2 wird die Rotation des Greiferkopfes beendet und es erfolgt ein Öffnen des Greiferkopfes 4 bis mit der vollkommen eingefahrenen Druck-/Dreheinrichtung 3 ein maximales Öffnen des Greiferkopfes 4 erfolgt ist. Ein Nachlassen des Drucks auf die Druck-/Dreheinrichtung 3 bewirkt ein Schließen des Greiferkopfes 4, wobei ein weiteres Nachlassen des Drucks nach dem Schließen des Greiferkopfes 4 eine Rotation desselben bewirkt, bis die Druck-/Dreheinrichtung 3 wieder in der Ausgangsposition ist. Mit anderen Worten, das Eindrücken der Druck-/Dreheinrichtung 3 in den Korpus 2 bewirkt zuerst eine Rotation des Greiferkopfes 4 mit anschließendem Öffnen desselben, während das Herausfahren der Druck-/Dreheinrichtung 3 aus dem Korpus 2 durch Nachlassen des Drucks zuerst ein Schließen des Greiferkopfes 4 mit einer nachfolgenden Rotation des Greiferkopfes 4 bewirkt.

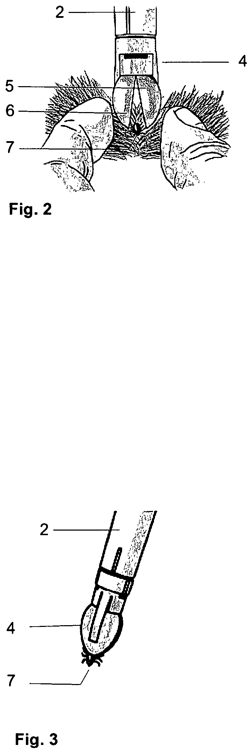

Fig. 2 beschreibt schematisch das Erfassen einer Zecke 7 mit dem geöffneten Greiferkopf 4, der in dieser Darstellung eine linke Greifbacke 5 und eine rechte Greifbacke 6 aufweist.

Dargestellt in Fig. 2 ist eine Zecke 7, die sich im Haarbereich eines Menschen oder eines Tieres in der Haut festgesaugt hat. Um diese Zecke 7 entfernen zu können, wird Zecke 7 im Haarbereich durch die Finger einer Person freigelegt. Dann wird der Greiferkopf 4 mit denen geöffneten Backen 5, 6 über die Zecke 7 platziert, wobei hier der Zeckengreifer 1 schematisch durch einen Teil des Korpus 2 dargestellt ist. Ist dies erfolgt, so wird der Greiferkopf 4 geschlossen und die Zecke 7 von den Greifbacken 5, 6 des Greiferkopfes 4 ergriffen. Das Schließen des Greiferkopfes 4 erfolgt durch Nachlassen des Drucks auf die Druck-/Dreheinrichtung 3, sodass sich die Druck-/Dreheinrichtung wieder aus dem Korpus 2 hinaus bewegt. Ist das Schließen des Greiferkopfes 4 beendet und die Zecke ergriffen, so bewirkt ein weiteres Nachlassen des Drucks auf die Druck-/Dreheinrichtung 3 eine Rotation des Greiferkopfes 4, wodurch die Zecke 7 aus der Haut des Patienten entfernt wird.

In der Fig. 3 ist schematisch der Endzustand nach dem Entfernen der Zecke 7 aus der Haut des Patienten dargestellt. Nachdem die Druck-/Dreheinrichtung 3 völlig entspannt ist und die ausgefahrene Endposition in dem Korpus 2 des Zeckengreifers 1 eingenommen hat, ist die Zecke 7 in den Greifbacken des Greiferkopfes 4 fixiert, wobei der Zeckengreifer 1 in der Figur schematisch durch einen Teil des Korpus 2 dargestellt ist.

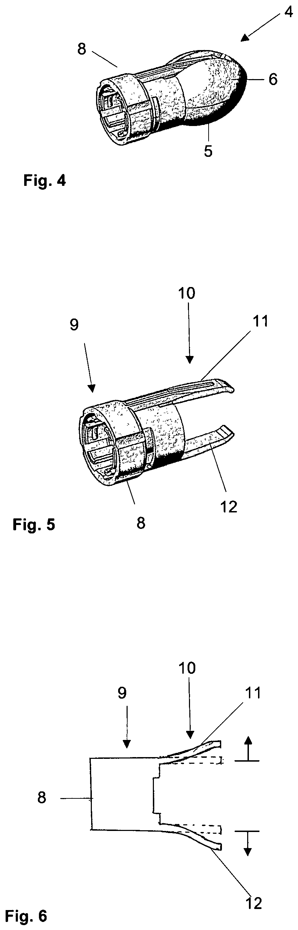

Fig. 4 zeigt in perspektivischer Darstellung den Greiferkopf 4 eines Zeckengreifers 1, der eine Halterung 8 aufweist, in welcher die beiden Greifbacken 5, 6 angeordnet sind. Dabei ist in der Anordnung der Fig. 4 eine der Greifbacken 5, 6 oben und die andere unten.

In Fig. 5 ist die Halterung 8 in größerem Detail und ohne die beiden Greifbacken 5, 6 dargestellt. Dabei umfasst die Halterung 8 eine Basis 9, die zur Anordnung des Greiferkopfes 4 mittels einer geeigneten Klemmeinrichtung (nicht dargestellt) an die Drück-/Dreheinrichtung 3 dient. Ferner schließt sich an die Basis 9 der Halterung 8 eine Schließfeder 10 an, wobei die Schließfeder 10 ein erstes Federelement 11 und ein zweites Federelement 12 aufweist. Die Federelemente 11, 12 liegen einander gegenüber und sind so ausgebildet, dass sie jeweils in eine entsprechende Aussparung der jeweiligen Greifbacke 5, 6 eingreifen, wie dies der Fig. 4 entnommen werden kann.

Die Eigenschaften der Schließfeder 10 werden durch verschiedene Faktoren wie etwa Materialauswahl, Zusätze zum Material, Formteil- und Werkzeugauslegung, Verarbeitungs- oder Umweltbedingungen beeinflusst. Durch Bestimmung der erforderlichen Materialkonstanten und Berechnungen zur Ermittlung von Feder- und Fügekräften beim Öffnen und Schließen des Greiferkopfes 4 kann ein exaktes Öffnungs- und Schließverhalten sicher gestellt werden. Das Schließverhalten lässt sich aus der Kraftkurve ermitteln, die sich aus der Schließkraft des Greiferkopfes 4 in Abhängigkeit von der Öffnungsweite ergibt und ist entscheidend dafür, dass die Zecke zwar gehalten, aber nicht übermäßig gedrückt wird. Das Öffnungsverhalten ergibt sich aus dem erforderlichen Druck auf den Druckkopf und der maximalen Spreizweite, die nach einer 720°-Rotation erreicht wird. Die Vorspannung zum Zeitpunkt des Fügens der beiden Bauteile beträgt 1,1 N pro Federelement 11, 12. Durch Spannungsrelaxation wird die Vorspannung mit der Zeit abgebaut, der Langzeitwert für ca. 10 Jahre beträgt 0,5 N pro Federelement 11, 12.

Fig. 6 zeigt das Prinzip der Schließfeder 10 in schematischer Darstellung. Die Federelemente 11, 12 der Schließfeder 10 sind einstückig mit der Basis 9 der Halterung 8 ausgelegt. Dabei sind die Federelemente 11, 12 aus der gestrichelt dargestellten Ruhestellung nach außen in Richtung der Pfeile beweglich. Mit anderen Worten, im gestrichelt dargestellten entspannten Zustand drücken die Federelemente 11, 12 die Greifbacken (nicht dargestellt) aufeinander. Werden die Federelemente 11, 12 durch die Drück-/Dreheinrichtung 3 (nicht dargestellt) nach außen gedrückt, so öffnen sich die Greifbacken 4, 5 des Greiferkopfes 4.

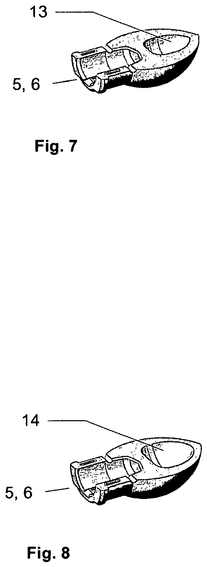

Fig. 7 zeigt eine Greifbacke 5, 6 in perspektivische Draufsicht, die in der Fig. 5 dargestellten Halterung 8 angeordnet ist. Dabei weist die dargestellte Greifbacke 5, 6 eine kleine Kavität 13 mit verstärkter Wand zum Erfassen kleiner und kleinster Zecken auf.

Fig. 8 schließlich zeigt eine weitere Version der Greifbacke 5, 6 mit einer großen Kavität 14 zur Aufnahme großer Zecken.

Die Formgebung der Greifbacken 5, 6 berücksichtigt die Morphologie der Zecke. Da Zecken auf der Menschenhaut oft frühzeitig bemerkt werden, sind hier eher kleinere Zecken zu entfernen. Im unübersichtlichen Tierfell werden Zecken naturgemäß später entdeckt, so dass beim Tier tendenziell größere Zecken zu entfernen sind. Daher kommen bei Mensch und Tier unterschiedlich geformte Greifbacken mit unterschiedlich großen Kavitäten 13, 14 zum Einsatz. Ziel ist es, den Druck auf die Speicheldrüsen, in denen sich das FSME-Virus befinden kann, oder den Druck auf den Darm, in dem sich Borrelien und andere pathogene Bakterien befinden können, so gering wie möglich zu halten.

Kleine Zecken, d.h. Zecken < 2 mm, zum Beispiel Nymphen oder sogenannte "Babyzecken" werden als Ganzes von den Greifbacken umschlossen. Sie "verschwinden" in der Wandstärke der Greifspitze. Darum ist der Einsatz eines weichen Materials wichtig.

Mittlere Zecken, d.h. Zecken 2 mm bis 4 mm, werden am Kopf und proximalem Rumpf von der Spitze des Greiferkopfes erfasst. Der Hinterleib der Zecke liegt in der Kavität.
Große Zecken, d.h. Zecken > 4 mm, werden wie die mittleren Zecken am Kopf und proximalem Rumpf von der Spitze des Greiferkopfes erfasst, aber der größte Teil des Körpers liegt in der Kavität. Diese sollte entsprechend groß sein, damit der Druck auf den Zeckendarm klein ist.

Mit anderen Worten, für kleine Zecken eignet sich der in Fig. 7 dargestellte Typ 1 mit kleiner Kavität 13, für größere Zecken eignet sich der in Fig. 8 dargestellte Typ 2 des Greiferkopfes 4 mit großer Kavität 14.

Ferner müssen die Greifbacken 5, 6 aus einem Material gefertigt sein,welches
- durch seine Eigenelastizität die Zecke nicht zu stark drückt, und
- durch seine Oberflächenbeschaffenheit wenig Reibungsverluste bei der Übertragung der Rotationskraft auf die Zecke mit sich bringt.

Diese Materialanforderungen werden von TPE (Thermoplastisches Elastomer) erfüllt. TPE gilt als Alternativwerkstoff zu Silikon und verfügt über exzellente mechanische Eigenschaften, denn es ist extrem flexibel, hat eine glatte Oberfläche, ist UV- und witterungsbeständig, besitzt ein gutes Rückstellvermögen und ist in einem weiten Temperatureinsatzbereich (- 40° C bis +120°C) einsetzbar. TPE ist dynamisch bis 100°C belastbar. Ferner ist TPE konform mit den Vorschriften der FDA (Food and Drug Administration) und recyclingfähig. Verschiedene Härten kommen bei der Herstellung der Greifbacken zum Einsatz, zum Beispiel 44, 55 und 65 Shore.

Zusammenfassend erfüllen die Greifbacken des erfindungsgemäßen Greifkopfes 4 bzw. des Zeckengreifers 1 die folgenden Anforderungen:
- der Greifkopf lässt sich so weit öffnen, dass auch große Zecken gefasst werden können,
- der Greifkopf lässt sich exakt schließen, dass auch Nymphen von einer Größe kleiner als 1 mm erfasst werden,
- die Zecke wird möglichst wenig gequetscht,
- der Greifkopf rotiert zum Herausdrehen der Zecke gleichmäßig, und
- das exakte Öffnungs- und Schließverhalten des Greifkopfes wird beibehalten, selbst wenn dieser sehr oft nacheinander geöffnet und geschlossen wird, bzw. lange Zeit nicht betätigt wird, und einem Temperaturbereich von 5 bis 50 ° Celsius ausgesetzt ist.

Ferner können die Greifbacken eine haftende Oberfläche aufweisen, so dass die Zecke an der Oberfläche haften bleibt. Eine Abtötung der Zecke 7 kann mittels Elektrizität, Hitze, Kälte oder chemischen Reaktionen nach Entfernung der Zecke aus der Haut erfolgen. Schließlich kann das Material der Greifbacken transparent sein, um die Sicht auf das zu entfernende Objekt zu verbessern, wobei zusätzlich die Anbringung einer Lichtquelle und/oder Lupe an den Zeckengreifer insbesondere bei kleinen Zecken sinnvoll sein kann.

### Bezugszeichenliste

- 1: Zeckengreifer
- 2: Korpus
- 3: Drück-/Dreheinrichtung
- 4: Greiferkopf
- 5: Greifbacke
- 6: Greifbacke
- 7: Zecke
- 8: Halterung
- 9: Basis
- 10: Schließfeder
- 11: Federelement
- 12: Federelement
- 13: kleine Kavität
- 14: große Kavität

## Patentansprüche

1. Greiferkopf (4) eines Zeckengreifers (1), wobei der Greiferkopf zum Ergreifen und Fixieren einer Zecke (7) dient, mit zwei gegenüberliegenden Greifbacken (5, 6), wobei der Greiferkopf (4) einen Zustand mit geöffneten Greifbacken und Zustand mit geschlossenen Greifbacken einnehmen kann,
wobei die Greifbacken (5, 6) so ausgestaltet sind, dass sie jeweils eine gekrümmte Außenseite und eine plane Innenseite aufweisen, die Schließfeder (10) auf die beiden Greifbacken (5, 6) wirkt, und im geschlossenen Zustand des Greiferkopfes (4) die planen Innenseiten der beiden Greifbacken (5, 6) aufeinander liegen, so dass ein geschlossener Körper gebildet wird,
**gekennzeichnet durch**
eine Halterung (8) mit einer Schließfeder (10) zur Aufnahme und zum Schließen der beiden Greifbacken (5,6), wobei die Schließfeder (10) zwei gegenüber angeordnete Federelemente (11, 12) aufweist, jede Greifbacke (5, 6) auf Ihrer Außenseite eine Aussparung aufweist, und
die Federelemente (11, 12) in die Aussparungen der Greifbacken (5, 6) eingreifen.

2. Greiferkopf (4) nach Anspruch 1, **dadurch gekennzeichnet, dass** die beiden Greifbacken (5, 6) jeweils eine innere Kavität (13, 14) aufweisen.

3. Greiferkopf (4) nach Anspruch 1 oder 2, **dadurch gekennzeichnet, dass** die die Federelemente (11, 12) der Schließfeder (10) eine vorgegebene Vorspannung aufweisen, wodurch der Schluss der Greifbacken (5, 6) im geschlossenen Zustand des Greiferkopfes (4) bewirkt wird.

4. Greiferkopf (4) nach einem der vorangegangenen Ansprüche, **dadurch gekennzeichnet, dass** die Federkonstante der Schließfeder (10) so gewählt wird, dass keine übermäßige Quetschung der zu entfernenden Zecke (7) erfolgt.

5. Greiferkopf (4) nach einem der vorangegangenen Ansprüche, **dadurch gekennzeichnet, dass** die Greifbacken aus einem thermoplastischen Elastomer bestehen.

6. Greiferkopf (4) nach einem der vorangegangenen Ansprüche, **dadurch gekennzeichnet, dass** die Halterung (8) des Greiferkopfes eine Einrichtung zur lösbaren Fixierung des Greiferkopfes (4) an dem Zeckengreifer (1) aufweist.

7. Zeckengreifer (1) zum Ergreifen und Fixieren einer Zecke (7) mit einem länglichen Korpus (2) und einer in dem Korpus (2) angeordneten Drück-/Dreheinrichtung (3) und einem Greiferkopf (4), wobei der Greiferkopf (4) durch die Drück-/Dreheinrichtung in Rotation versetzt sowie geöffnet und geschlossen werden kann,
**dadurch gekennzeichnet, dass**
der Zeckengreifer einen Greiferkopf nach einem der vorangegangenen Ansprüche aufweist.

8. Zeckengreifer (1) nach Anspruch 7, **dadurch gekennzeichnet, dass** die Drück-/Dreheinrichtung (3) des Zeckengreifers an einem Ende als Druckstift ausgebildet ist und an dem anderen Ende eine Aufnahmevorrichtung zum lösbaren Befestigen und Fixieren des Greiferkopfes (4) aufweist.

9. Zeckengreifer (1) nach Anspruch 8, **dadurch gekennzeichnet, dass** mit dem Einfahren des Druckstiftes der Drück-/Dreheinrichtung (3) in den Korpus (2) der Greiferkopf (4) zuerst in Rotation versetzt wird,
kurz vor Erreichen der komplett eingefahrenen Position die Rotation beendet und der Greiferkopf geöffnet wird,
mit dem Ausfahren des Druckstiftes aus dem Korpus (2) der Greiferkopf (4) geschlossen wird, und
der Greiferkopf (4) bei einem weiteren Ausfahren des Druckstiftes aus dem Korpus (2) in Rotation versetzt wird, bis der vollkommen ausgefahrenen Zustand des Druckstiftes aus dem Korpus (2) erreicht ist.

## Claims

1. A gripper head (4) for grabbing and fixing a tick (7), comprising two mutually facing gripping jaws (5, 6), wherein the gripper head (4) can adopt a state with open gripping jaws and a state with closed gripping jaws,
wherein the gripping jaws (5, 6) are configured such that they respectively have a curved outer side and a flat inner side, the closing spring (10) acts on the two gripping jaws (5, 6), and, in the closed state of the gripper head (4), the flat inner sides of the two gripping jaws (5, 6) lie one upon the other, so that a closed body is formed
**characterized by**
a holder (8) having a closing spring (10) for receiving the two gripping jaws (5, 6),wherein
the closing spring (10) has two mutually facing spring elements (11, 12), each gripping jaw (5, 6) has on its outer side a recess, and the spring elements (11, 12) engage in the recesses of the gripping jaws (5, 6).

2. The gripper head (4) as claimed in claim 1, **characterized in that** the two gripping jaws (5, 6) respectively have an inner cavity (13, 14).

3. The gripper head (4) as claimed in claim 1 or 2, **characterized in that** the spring elements (11, 12) of the closing spring (10) have a predefined preload, whereby the closure of the gripping jaws (5, 6) in the closed state of the gripper head (4) is effected.

4. The gripper head (4) as claimed in one of the preceding claims,
**characterized in that** the spring constant of the closing spring (10) is chosen such that no excessive squeezing of the tick (7) to be removed takes place.

5. The gripper head (4) as claimed in one of the preceding claims,
**characterized in that** the gripping jaws consist of a thermoplastic elastomer.

6. The gripper head (4) as claimed in one of the preceding claims,
**characterized in that** the holder (8) of the gripper head has a device for detachably fixing the gripper head (4) to a tick gripper (1).

7. A tick gripper (1) for grabbing and fixing a tick (7), comprising an elongate main body (2) and a pressing/rotating device (3) disposed in the main body (2), and a gripper head (4), wherein the gripper head (4) can be set in rotation by the pressing/rotating device, and opened and closed,
**characterized in that**
the tick gripper has a gripper head as claimed in one of the preceding claims.

8. The tick gripper (1) as claimed in claim 7, **characterized in that** the pressing/rotating device (3) of the tick gripper is configured at one end as a pressure pin and has at the other end a receiving fixture for the detachable fastening and fixing of the gripper head (4).

9. The tick gripper (1) as claimed in claim 8, **characterized in that,**
with the retraction of the pressure pin of the pressing/rotating device (3) into the main body (2), the gripper head (4) is firstly set in rotation, shortly before reaching of the completely retracted position, the rotation is ended and the gripper head opened,
with the extension of the pressure pin from the main body (2), the gripper head (4) is closed and, upon a further extension of the pressure pin from the main body (2), the gripper head (4) is set in rotation until the fully extended state of the pressure pin from the main body (2) is reached.

## Revendications

1. Tête de préhension (4) d'un dispositif de préhension de tique (1), la tête de préhension servant à saisir et à fixer une tique (7), comprenant deux mâchoires de préhension (5, 6) en regard, la tête de préhension (4) pouvant présenter un état dans lequel les mâchoires de préhension sont ouvertes et un état dans lequel les mâchoires de préhension sont fermées,
les mâchoires de préhension (5, 6) étant conçues de telle sorte qu'elles présentent respectivement un côté extérieur incurvé et un côté intérieur plan, un ressort de fermeture (10) agissant sur les deux mâchoires de préhension (5, 6) et, à l'état fermé de la tête de préhension (4), les côtés intérieurs plans des deux mâchoires de préhension (5, 6) reposant l'un sur l'autre de manière à former un corps fermé,
**caractérisée par**
un dispositif de retenue (8) comprenant un ressort de fermeture (10) servant à recevoir et à fermer les deux mâchoires de préhension (5, 6),
le ressort de fermeture (10) comprenant deux éléments de ressort (11, 12) disposés en regard, chaque mâchoire de préhension (5, 6) comprenant un évidement sur son côté extérieur, et les éléments de ressort (11, 12) venant en prise dans les évidements des mâchoires de préhension (5, 6).

2. Tête de préhension (4) selon la revendication 1, **caractérisée en ce que** les deux mâchoires de préhension (5, 6) comprennent respectivement une cavité intérieure (13, 14).

3. Tête de préhension (4) selon la revendication 1 ou 2, **caractérisée en ce que** les éléments de ressort (11, 12) du ressort de fermeture (10) présentent une précontrainte prédéfinie, de sorte que la fermeture des mâchoires de préhension (5, 6) soit provoquée à l'état fermé de la tête de préhension (4).

4. Tête de préhension (4) selon l'une quelconque des revendications précédentes, **caractérisée en ce que** la constante de rappel du ressort de fermeture (10) est sélectionnée de manière à ce qu'aucun écrasement excessif de la tique (7) à retirer ne soit effectué.

5. Tête de préhension (4) selon l'une quelconque des revendications précédentes, **caractérisée en ce que** les mâchoires de préhension sont constituées d'un élastomère thermoplastique.

6. Tête de préhension (4) selon l'une quelconque des revendications précédentes, **caractérisée en ce que** le dispositif de retenue (8) de la tête de préhension comprend un dispositif permettant la fixation libérable de la tête de préhension (4) au dispositif de préhension de tique (1).

7. Dispositif de préhension de tique (1) permettant de saisir et de fixer une tique (7), comprenant un corps allongé (2) et un dispositif de compression/rotation (3) disposé dans le corps (2) et une tête de préhension (4), la tête de préhension (4) pouvant être mise en rotation par le dispositif de compression/rotation ainsi qu'ouverte et fermée,
**caractérisé en ce que**
le dispositif de préhension de tique comprend une tête de préhension selon l'une quelconque des revendications précédentes.

8. Dispositif de préhension de tique (1) selon la revendication 7,
**caractérisé en ce que** le dispositif de compression/rotation (3) du dispositif de préhension de tique est réalisé, à une extrémité, sous forme de goupille de pression et comprend, à l'autre extrémité, un dispositif de réception servant à assujettir de manière libérable et à fixer la tête de préhension (4).

9. Dispositif de préhension de tique (1) selon la revendication 8,
**caractérisé en ce que**
lors de la rentrée de la goupille de pression du dispositif de compression/rotation (3) dans le corps (2), la tête de préhension (4) est tout d'abord mise en rotation,
juste avant que la position complètement rentrée ne soit atteinte, la rotation est terminée et la tête de préhension est ouverte,
lors de la sortie de la goupille de pression hors du corps (2), la tête de préhension (4) est fermée, et
la tête de préhension (4) est, lors d'une sortie supplémentaire de la goupille de pression hors du corps (2), mise en rotation jusqu'à ce que l'état complètement sorti de la goupille de pression hors du corps (2) soit atteint.
